# FASCICULE DE BREVET EUROPEEN

(11) **EP 3 544 660 B1**
(45) Date de publication et mention de la délivrance du brevet: **09.12.2020**
(21) Numéro de dépôt: 17797392.2
(22) Date de dépôt: 18.10.2017
(51) Int. Cl.: A61M 5/20, A61M 5/32

(54) **DISPOSITIF D'INJECTION PRÉSENTANT UN DISPOSITIF DE RETRAIT DE CAPUCHON DE SERINGUE AMÉLIORÉ**
INJEKTIONSVORRICHTUNG MIT VERBESSERTER SPRITZENKAPPENENTFERNUNG
INJECTION DEVICE HAVING AN IMPROVED SYRINGE CAP REMOVAL

(30) Priorité: 23.11.2016 FR 1661383
(43) Date de publication de la demande: 02.10.2019
(73) Titulaire: Nemera La Verpilliere, 38290 La Verpilliere (FR)
(72) Inventeur: DUGAND, Pascal, 38780 Estrablin (FR); STAMP, Kevin, Chapeltown, Sheffield S35 1AR (GB)
(74) Mandataire: LLR
(86) Numéro de dépôt international: PCT/FR2017/052865
(87) Numéro de publication internationale: WO 2018/096231

(56) Documents cités:
- EP-A1- 2 745 866
- EP-A1- 2 878 322
- WO-A1-2014/091153
- WO-A1-2015/110532
- WO-A1-2016/193374
- GB-A- 2 438 593
- US-A1- 2016 089 495
- US-A1- 2016 220 765

## Description

La présente invention concerne les ensembles d'injection comprenant un dispositif d'assistance au fonctionnement d'une seringue d'injection et une seringue d'injection logée dans ce dispositif d'assistance.

Dans le domaine médical par exemple, une seringue d'injection permet d'injecter un médicament liquide dans le corps d'un patient. La seringue d'injection comprend habituellement un corps de seringue portant une aiguille d'injection, un piston logé, dans le corps de la seringue, délimitant une chambre contenant le médicament à injecter, et une tige de manoeuvre de ce piston.

Il est courant que ce soit un tiers qui injecte le médicament dans le corps du patient au moyen d'une seringue contenant ce médicament.

Or, il convient d'éviter autant que possible que le tiers ne se pique involontairement avec l'aiguille de la seringue lorsqu'il manipule cette dernière.

On a donc développé dans l'état de la technique, par exemple conformément à l'enseignement de WO 2015/044561, un premier type de dispositif d'assistance au fonctionnement d'une seringue d'injection permettant d'empêcher ou de limiter les accidents de manipulation d'une seringue, par exemple en masquant automatiquement, juste après l'injection, l'aiguille de la seringue.

Le document WO 2015/110532 A1 décrit un dispositif tel que celui décrit dans le préambule de la revendication 1.

Parfois, c'est le patient lui-même qui réalise l'injection sur une partie de son corps. C'est le cas par exemple des patients atteints d'arthrite rhumatoïde, de sclérose en plaque, de diabète ou subissant un choc anaphylactique en cas d'allergie.

Dans ce cas, comme lorsque l'injection est réalisée par un tiers, il convient d'éviter autant que possible que le patient ne se pique involontairement avec l'aiguille de la seringue lorsqu'il manipule cette dernière. Mais il faut également faciliter la manoeuvre de la seringue du fait que le patient qui réalise lui-même l'injection peut-être affaibli ou ressentir une certaine appréhension à réaliser l'injection.

On a donc développé dans l'état de la technique un second type de dispositif d'assistance au fonctionnement d'une seringue d'injection permettant, comme dans le cas précédent, d'éviter les accidents de manipulation de la seringue, mais également rendant automatiques certaines étapes de fonctionnement de la seringue, notamment l'enfoncement de l'aiguille de la seringue dans le corps du patient et l'enfoncement du piston de la seringue dans le corps de cette seringue.

Les dispositifs d'assistance au fonctionnement d'une seringue d'injection évoqués plus haut comportent généralement un support de seringue dans lequel est logée une seringue pré-remplie. Avant utilisation, l'aiguille d'injection de la seringue est recouverte d'un capuchon de protection amovible. Pour éviter un accident de manipulation dès le retrait du capuchon par l'utilisateur, les dispositifs d'assistance au fonctionnement d'une seringue d'injection évoqués plus haut comportent habituellement un dispositif de retrait du capuchon relié de façon amovible au support de seringue, permettant un retrait du capuchon dans de bonnes conditions de sécurité. Un tel dispositif de retrait de capuchon, connu par exemple de WO 2015/044561, comprend :
- un organe de préhension, destiné à être manoeuvré par un utilisateur pour retirer le capuchon de l'aiguille, et
- des extracteurs coopérant avec le capuchon lors du retrait du capuchon.

Avant utilisation, le dispositif de retrait du capuchon est relié de façon amovible au support de seringue.

Le dispositif d'assistance décrit dans WO 2015/044561 comprend des premier et second organes complémentaires de commande de chaque extracteur déplaçables l'un par rapport à l'autre selon un mouvement relatif de coopération entre eux, dit mouvement relatif d'accrochage. Ce mouvement relatif d'accrochage permet de faire évoluer chaque extracteur entre une configuration de référence escamotée par rapport au capuchon et une configuration de coopération avec le capuchon.

Les premier et second organes complémentaires de commande décrits dans WO 2015/044561 sont respectivement portés par le support de seringue et par l'organe de préhension.

Avant son retrait, le capuchon de protection de l'aiguille est maintenu emboité sur une partie complémentaire du corps de seringue, par frottement.

Par ailleurs, le mouvement relatif d'accrochage se fait à l'encontre de forces de frottement notamment entre les organes complémentaires de commande d'extracteur.

Enfin, dans le dispositif d'assistance décrit dans WO 2015/044561, le mouvement relatif d'accrochage est couplé au mouvement de manoeuvre appliqué par l'utilisateur à l'organe de préhension pour retirer le capuchon de l'aiguille.

Par conséquent, dans WO 2015/044561, du fait de ce couplage de mouvements, le mouvement de manoeuvre appliqué par l'utilisateur à l'organe de préhension pour retirer le capuchon de l'aiguille se fait à l'encontre :
- d'une part, des forces de frottement entre les organes complémentaires de commande d'extracteur, et
- d'autre part, des forces de frottement maintenant le capuchon de protection emboité sur une partie complémentaire du corps de seringue.

Or, il est habituellement imposé que la force nécessaire pour séparer le capuchon de protection de l'aiguille soit relativement limitée afin que l'utilisateur puisse enlever le capuchon facilement.

Du fait que dans WO 2015/044561, le mouvement de manoeuvre appliqué par l'utilisateur à l'organe de préhension pour retirer le capuchon de l'aiguille se fait à l'encontre, à la fois, des forces de frottement entre les organes complémentaires de commande d'extracteur, et des forces de frottement maintenant le capuchon de protection emboité, il est difficile de respecter la limite d'effort imposée pour un retrait facile du capuchon par un utilisateur.

Par ailleurs, chaque extracteur est généralement formé par un bras élastique s'étendant sensiblement longitudinalement dans une partie en forme générale de manchon de l'organe de préhension. Ce bras élastique forme un levier déformable entre les configurations de référence escamotée et de coopération de l'extracteur. Une extrémité de résistance du levier, en forme de crochet, est destinée, lors du mouvement de manoeuvre appliqué par l'utilisateur à l'organe de préhension pour retirer le capuchon de l'aiguille, à venir prendre prise avec un bord d'extrémité du capuchon. Or, ce bord d'extrémité du capuchon est dans certains cas peu dégagé si bien que l'extracteur risque de ne pas se mettre en prise avec le capuchon lorsqu'il est entrainé par le mouvement de manoeuvre appliqué par l'utilisateur à l'organe de préhension pour retirer le capuchon de l'aiguille.

L'invention a pour but de proposer un dispositif d'assistance au fonctionnement d'une seringue d'injection muni d'un dispositif de retrait de capuchon facile à manipuler, c'est-à-dire ne requérant qu'un effort limité de la part d'un utilisateur pour retirer le capuchon.

À cet effet, l'invention a pour objet un dispositif d'assistance au fonctionnement d'une seringue d'injection comprenant un corps de seringue portant une aiguille d'injection recouverte d'un capuchon de protection amovible, le dispositif d'assistance comprenant :
- un support de seringue dans lequel le corps de seringue est destiné à être logé, et
- un dispositif de retrait du capuchon de protection, relié de façon amovible au support de seringue, comprenant :
   - un organe de préhension, destiné à être manoeuvré par un utilisateur pour retirer ce capuchon de protection de l'aiguille d'injection, et
   - au moins un extracteur coopérant avec le capuchon de protection lors du retrait du capuchon de protection,
   caractérisé en ce que le dispositif de retrait comprend des premier et second organes complémentaires de commande de l'extracteur déplaçables l'un par rapport à l'autre selon un mouvement relatif de coopération entre eux, dit mouvement relatif d'accrochage, pour faire évoluer l'extracteur entre une configuration de référence escamotée par rapport au capuchon de protection et une configuration de coopération avec le capuchon de protection, le mouvement relatif d'accrochage étant indépendant du mouvement de manoeuvre appliqué par l'utilisateur à l'organe de préhension pour retirer le capuchon de protection de l'aiguille d'injection.

Du fait que le mouvement relatif d'accrochage est indépendant du mouvement de manoeuvre appliqué par l'utilisateur à l'organe de préhension pour retirer le capuchon de l'aiguille, ces deux mouvements peuvent être réalisés à des moments différents.

Ainsi, le mouvement relatif d'accrochage permettant de faire évoluer l'extracteur entre ses configurations de référence escamotée par rapport au capuchon et de coopération avec le capuchon, pourra être réalisé pendant une étape d'assemblage du dispositif d'assistance au fonctionnement de la seringue d'injection ou bien pendant une étape d'assemblage d'un ensemble d'injection comprenant ce dispositif d'assistance et une seringue d'injection logée dans ce dispositif d'assistance.

On pourra alors, au cours de l'assemblage, vérifier que l'extracteur est bien en prise avec le capuchon. L'ensemble d'injection comprenant le dispositif d'assistance et la seringue d'injection logée dans ce dispositif d'assistance sera donc livré à l'utilisateur avec un extracteur en prise avec le capuchon, avant même que l'utilisateur manoeuvre l'organe de préhension pour retirer le capuchon de l'aiguille.

Par la suite, lorsque l'utilisateur manoeuvrera l'organe de préhension pour retirer le capuchon de l'aiguille, il n'aura plus qu'à vaincre essentiellement les forces de frottement maintenant le capuchon de protection emboîté sur la partie complémentaire du corps de la seringue.

On assure ainsi, grâce à l'invention, un retrait facile et sûr du capuchon par un utilisateur.

Suivant d'autres caractéristiques optionnelles du dispositif d'assistance au fonctionnement de la seringue d'injection et différents modes de réalisation de l'invention :
- l'organe de préhension comporte une partie proximale en forme générale de manchon, l'extracteur étant formé par un bras élastique s'étendant sensiblement longitudinalement dans la partie proximale et formant un levier du troisième genre déformable entre les configurations de référence escamotée et de coopération, l'extracteur étant muni d'une extrémité, dite de résistance, destinée à coopérer avec le capuchon de protection ;
- le dispositif de retrait comprend au moins deux extracteurs formant respectivement deux leviers du troisième genre, sensiblement parallèles entre eux, ces extracteurs étant munis chacun d'une extrémité, dite de résistance, destinée à coopérer avec le capuchon de protection, et d'une extrémité opposée à l'extrémité de résistance, dite d'appui, les extrémités d'appui des extracteurs étant reliées entre-elles ;
- le mouvement de manoeuvre appliqué par l'utilisateur à l'organe de préhension pour retirer le capuchon de protection de l'aiguille d'injection comprend une translation, le mouvement relatif d'accrochage étant parallèle à cette translation ;
- les premier et second organes complémentaires de commande de l'extracteur coopèrent entre eux par effet de rampe sous l'effet du mouvement relatif d'accrochage ;
- les premier et second organes complémentaires de commande de l'extracteur sont respectivement portés par l'organe de préhension et par l'extracteur ;
- l'extracteur est porté par un coulisseau monté coulissant en translation dans l'organe de préhension, les premier et second organes complémentaires de commande de l'extracteur étant respectivement solidaires en translation de l'organe de préhension et de l'extracteur ;
- le premier organe de commande de l'extracteur est ménagé sur une surface interne de la partie proximale et le deuxième organe de commande de l'extracteur est ménagé sur l'extracteur ;
- le support de seringue et le coulisseau comportent des butées complémentaires de positionnement de l'extracteur dans sa configuration de référence escamotée par rapport au capuchon de protection ;
- l'organe de préhension et le coulisseau comprennent des moyens complémentaires d'attelage entre eux autorisant entre eux le mouvement relatif d'accrochage ;
- l'organe de préhension et le coulisseau comprennent des moyens complémentaires de verrouillage entre eux activables à la fin du mouvement relatif d'accrochage ;
- l'extracteur est solidaire de l'organe de préhension, le premier organe de commande de l'extracteur étant porté par un organe de manoeuvre monté coulissant en translation dans l'organe de préhension et le second organe de commande de l'extracteur étant solidaire en translation de l'extracteur ;
- l'organe de préhension et le support de seringue comportent des butées complémentaires de positionnement de l'extracteur dans sa configuration de référence escamotée par rapport au capuchon de protection ;
- l'organe de préhension et le support de seringue comportent des moyens complémentaires de verrouillage entre eux libérables à l'initiation du mouvement de manoeuvre appliqué par l'utilisateur à l'organe de préhension pour retirer le capuchon de protection de l'aiguille d'injection ;
- le support de seringue comporte un corps externe de forme générale tubulaire et un manchon d'extrémité faisant saillie à une extrémité de ce corps externe,
   la butée portée par le support de seringue pour le positionnement de l'extracteur dans sa configuration de référence escamotée par rapport au capuchon de protection étant ménagée sur l'extrémité du corps externe, et
   les moyens complémentaires de verrouillage de l'organe de préhension et du support de seringue étant ménagés sur l'organe de préhension et le manchon d'extrémité ;

   - l'organe de préhension et l'organe de manoeuvre portant le premier organe de commande de l'extracteur comprennent des moyens complémentaires de verrouillage entre eux libérables à l'initiation du mouvement relatif d'accrochage ;
   - l'organe de préhension et l'organe de manoeuvre portant le premier organe de commande de l'extracteur comprennent des moyens complémentaires de verrouillage entre eux activables à la fin du mouvement relatif d'accrochage.

L'invention a également pour objet un ensemble d'injection comprenant un dispositif d'assistance au fonctionnement d'une seringue d'injection et une seringue d'injection logée dans ce dispositif d'assistance, caractérisé en ce que le dispositif d'assistance au fonctionnement de la seringue d'injection est selon l'invention.

L'invention sera mieux comprise à la lecture de la description qui va suivre donnée uniquement à titre d'exemple et faite en se référant aux dessins annexés dans lesquels :
- les figures 1 à 4 sont des vues en perspective d'un ensemble d'injection comprenant un dispositif d'assistance au fonctionnement d'une seringue d'injection selon un premier mode de réalisation de l'invention, dans différentes configurations d'utilisation ou d'assemblage;
- la figure 5 est une vue en perspective d'un dispositif de retrait de capuchon de protection, équipant le dispositif d'assistance au fonctionnement illustré sur les figures précédentes ;
- la figure 6 est une vue en perspective d'un coulisseau monté dans le dispositif de retrait du capuchon de protection illustré à la figure 5 ;
- la figure 7 est une vue en coupe, suivant la ligne VII-VII de la figure 3, d'une partie distale du dispositif représenté sur les figures précédentes ;
- la figure 8 est une vue en coupe, suivant la ligne VIII-VIII de la figure 7, de la partie distale du dispositif représenté sur les figures précédentes ;
- la figure 9 est une vue en coupe, suivant la ligne IX-IX de la figure 1, de la partie distale du dispositif représenté sur les figures précédentes ;
- la figure 10 est une vue en coupe, suivant la ligne X-X de la figure 9, de la partie distale du dispositif représenté sur les figures précédentes ;
- les figures 11 à 13 sont des vues en perspective d'un ensemble d'injection comprenant un dispositif d'assistance au fonctionnement d'une seringue d'injection selon un second mode de réalisation de l'invention, dans différentes configurations d'utilisation ou d'assemblage ;
- les figures 14 et 15 sont des vues en perspective montrant respectivement un organe de préhension et un organe de manoeuvre d'un dispositif de retrait de capuchon de protection équipant le dispositif d'assistance selon le second mode de réalisation de l'invention ;
- la figure 16 est une vue en perspective éclatée du dispositif de retrait de capuchon de protection équipant le dispositif d'assistance au fonctionnement selon le second mode de réalisation de l'invention ;
- la figure 17 est une vue en coupe, suivant le plan XVII de la figure 12, d'une partie distale du dispositif d'assistance au fonctionnement selon le second mode de réalisation de l'invention ;
- la figure 18 est une vue en coupe, suivant la ligne XVIII-XVIII de la figure 17, de la partie distale du dispositif d'assistance au fonctionnement selon le second mode de réalisation de l'invention ;
- les figures 19 et 20 sont des vues en coupe, respectivement suivant les plans XIX et XX de la figure 11, de la partie distale du dispositif d'assistance au fonctionnement selon le second mode de réalisation de l'invention.

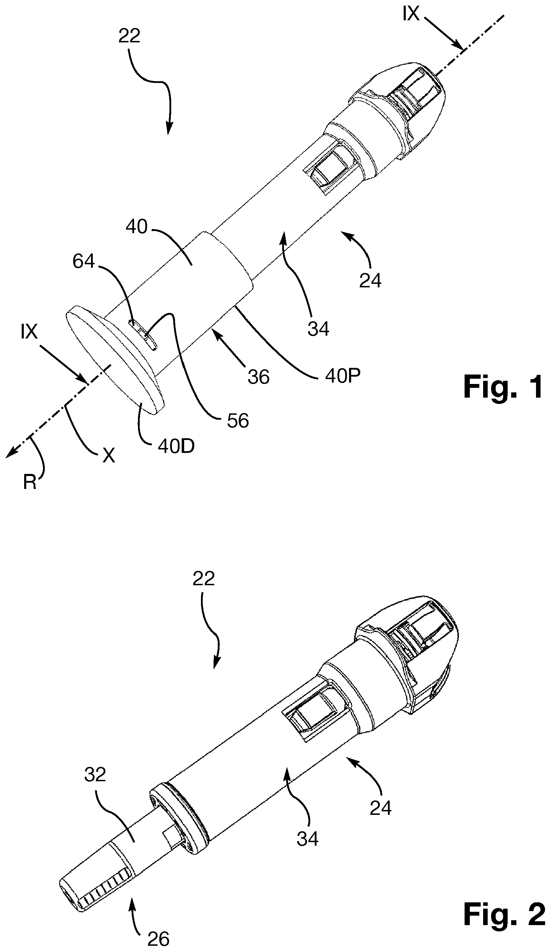

On a représenté sur les figures 1 à 10 un ensemble d'injection 22 de forme très générale de révolution autour d'un axe X.

L'ensemble d'injection 22 comprend un dispositif 24 d'assistance au fonctionnement d'une seringue d'injection, selon un premier mode de réalisation de l'invention. L'ensemble d'injection 22 comprend également une seringue d'injection 26 (visible notamment sur les figures 2, 4 et 7) logée dans ce dispositif d'assistance 24.

Dans l'exemple illustré, la seringue d'injection 26 permet d'injecter un médicament liquide dans le corps d'un patient.

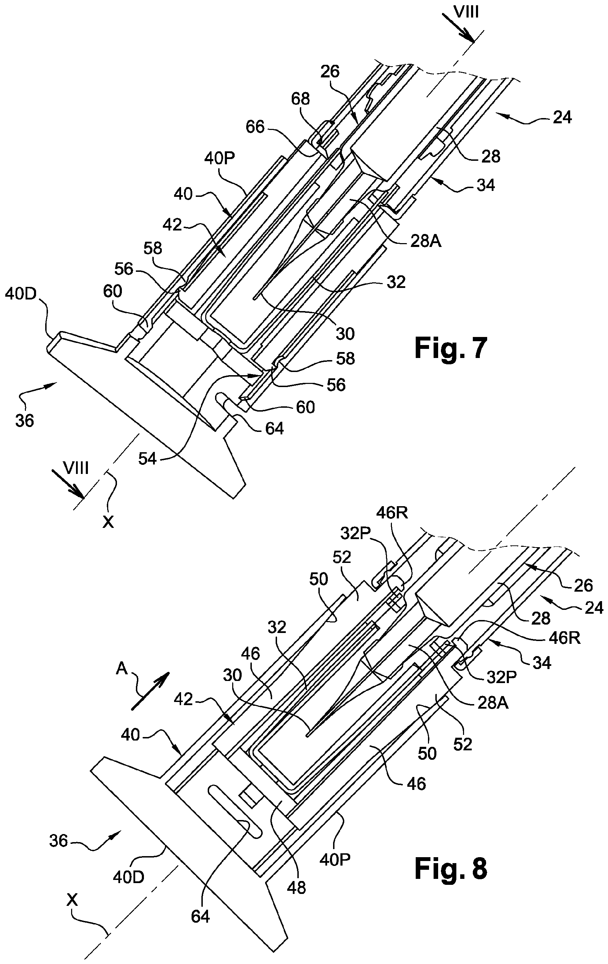

Comme cela est illustré notamment sur les figures 7 et 8, la seringue d'injection 26 comprend, de façon classique, un corps 28 de seringue portant une aiguille d'injection 30. Avant utilisation de l'ensemble d'injection 22, l'aiguille d'injection 30 est recouverte d'un capuchon de protection 32 amovible. Ce capuchon de protection 32, recouvrant l'aiguille d'injection 30, est maintenu emboîté sur une partie complémentaire 28A du corps 28 de seringue, par frottement.

La seringue d'injection 26 comprend également un piston (non représenté) logé dans le corps 28 de seringue et délimitant une chambre contenant le médicament à injecter. Le piston est classiquement relié à une tige de manoeuvre.

Le dispositif d'assistance 24 est du type décrit dans WO 2015/044561. Il permet d'empêcher ou de limiter les accidents de manipulation de la seringue d'injection 26, par exemple en masquant automatiquement, juste après l'injection, l'aiguille d'injection 30 de la seringue d'injection 26.

Dans ce qui suit, on qualifiera un élément du dispositif d'assistance 24 de proximal ou de distal selon qu'il est proche ou éloigné de la main de l'utilisateur actionnant ce dispositif d'assistance 24, lorsque l'utilisateur réalise une injection de médicament à l'aide du dispositif d'assistance 24.

Comme on peut le voir notamment sur les figures 1 à 4, 7 et 8, le dispositif d'assistance 24 comprend un support 34 de seringue, comprenant un ensemble d'éléments classiques, dans lequel le corps de seringue 28 est logé.

Le dispositif d'assistance 24 comprend également un dispositif 36 de retrait du capuchon de protection 32. Ce dispositif de retrait 36 est relié de façon amovible au support 34 de seringue.

Comme on peut le voir notamment sur les figures 7 à 10, le dispositif 36 de retrait du capuchon de protection 32 comprend un organe de préhension 40, destiné à être manoeuvré par un utilisateur pour retirer ce capuchon de protection 32 de l'aiguille d'injection 30, et un coulisseau 42 monté coulissant en translation, sensiblement parallèlement à l'axe X, dans l'organe de préhension 40.

L'organe de préhension 40 et le coulisseau 42 sont réalisés de préférence en plastique.

De préférence, le mouvement de manoeuvre appliqué par l'utilisateur à l'organe de préhension 40 pour retirer le capuchon de protection 32 de l'aiguille d'injection 30 correspond à une translation, sensiblement parallèle à l'axe X, réalisée dans le sens proximal vers distal illustré par la flèche R sur la figure 1.

En se référant notamment aux figures 1, 3 et 5, on voit que l'organe de préhension 40 comporte une partie proximale 40P, en forme générale de manchon, prolongée par une tête distale 40D dont la surface externe s'évase dans le sens proximal vers distal. Le coulisseau 42 est monté coulissant en translation dans la partie proximale 40P de l'organe de préhension 40.

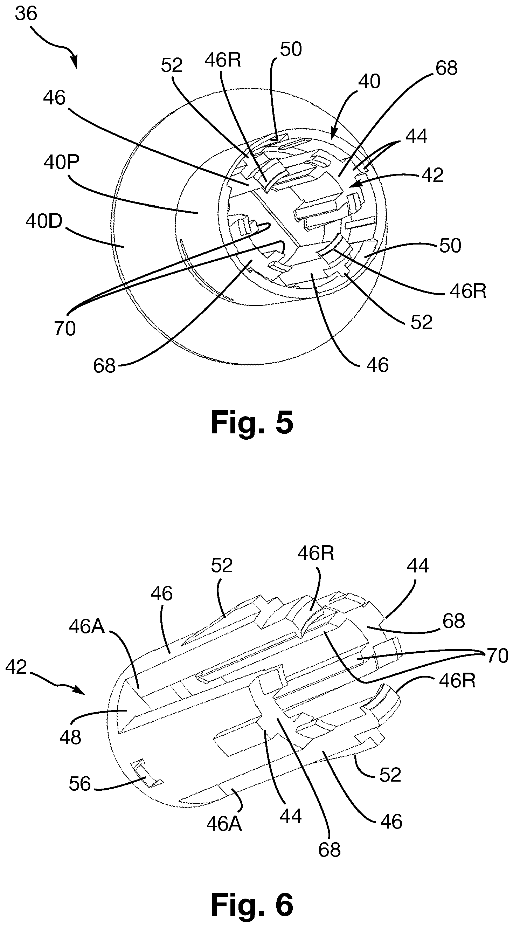

L'organe de préhension 40 et le coulisseau 42 comportent des moyens complémentaires 44 de guidage relatif en translation (visibles sur les figures 5 et 6), par exemple de type à rainure et languette.

Par ailleurs, le coulisseau 42 comprend deux extracteurs 46, sensiblement parallèles entre eux, destinés à coopérer avec le capuchon de protection 32 lors du retrait de ce capuchon de protection 32.

Plus particulièrement, les deux extracteurs 46 sont destinés à coopérer avec deux parties diamétralement opposées de la partie proximale 40P.

En variante, le coulisseau 42 pourrait ne comporter qu'un seul extracteur 46 ou bien plus de deux extracteurs 46, par exemple trois.

Comme cela apparaîtra plus en détail par la suite, chaque extracteur 46 est destiné à évoluer entre une configuration de référence escamotée par rapport au capuchon de protection 32 et une configuration de coopération avec ce capuchon de protection 32.

Chaque extracteur 46 est formé par un bras élastique s'étendant sensiblement longitudinalement dans la partie proximale 40P. Chaque extracteur 46 est rappelé élastiquement dans un état de repos qui correspond à sa configuration de référence escamotée.

On rappellera que l'on distingue habituellement trois genres de leviers suivant les positions relatives de l'axe de pivotement (point d'appui du levier) et des points d'application des forces motrice et résistante.

De préférence, chaque extracteur 46 forme un levier du troisième genre déformable entre les configurations de référence escamotée et de coopération. Ainsi, d'une part, chaque extracteur 46 est muni d'une extrémité, dite de résistance 46R, par exemple en forme de crochet, destinée à coopérer avec un bord d'extrémité proximal 32P du capuchon de protection 32. En variante, l'extrémité de résistance 46R de chaque extracteur 46 pourrait coopérer avec une autre surface du capuchon de protection 32. Ainsi, lorsque la seringue d'injection porte un capuchon de protection pourvu de fenêtres latérales, les extracteurs sont prévus de sorte que l'extrémité de résistance d'au moins un des extracteurs s'insère dans une des fenêtres de façon à agripper (coopérer avec) le bord de cette fenêtre en configuration de coopération. Selon un autre mode de réalisation non représenté, les extracteurs sont prévus plus courts que le capuchon de protection, la surface intérieure de chaque extracteur étant pourvue d'une surface d'agrippage destinée, en configuration de coopération, à agripper le capuchon de protection par friction. La surface d'agrippage peut être lisse, pourvue de relief ou d'un état de surface favorisant les frottements. D'autre part, chaque extracteur 46 est muni d'une extrémité, dite d'appui 46A, opposée à l'extrémité de résistance 46R, venue de matière avec une embase 48 formant une extrémité distale du coulisseau 42 (voir notamment figures 6 et 8). Ainsi, les extrémités d'appui 46A des extracteurs 46 sont reliées entre elles par l'embase 48 du coulisseau 42.

Pour chaque extracteur 46, le dispositif de retrait 36 comprend des premier 50 et second 52 organes complémentaires de commande de l'extracteur 46 (voir notamment figures 5, 6, 8 et 10) déplaçables l'un par rapport à l'autre selon un mouvement relatif de coopération entre eux, dit mouvement relatif d'accrochage. Ce mouvement relatif d'accrochage est indépendant du mouvement de manoeuvre appliqué par l'utilisateur à l'organe de préhension 40 lorsqu'il retire le capuchon de protection 32 de l'aiguille d'injection 30. Plus particulièrement, le mouvement relatif d'accrochage est parallèle au mouvement de translation appliqué par l'utilisateur lorsqu'il manoeuvre l'organe de préhension 40 pour retirer le capuchon de protection 32 de l'aiguille d'injection 30. Toutefois, en considérant le support 34 et le coulisseau 42 fixes, le mouvement relatif d'accrochage se fait en déplaçant l'organe de préhension 40 dans le sens opposé à celui représenté par la flèche R sur la figure 1. Le sens du mouvement relatif d'accrochage, dans les conditions évoquées ci-dessus, est représenté par la flèche A sur la figure 8.

De préférence, les premier 50 et second 52 organes complémentaires de commande de l'extracteur 46 coopèrent entre eux par effet de rampe sous l'effet du mouvement relatif d'accrochage. Ainsi, en se référant notamment aux figures 5 et 6, on voit que, pour chaque extracteur 46, le premier organe de commande est formé par un enfoncement 50 ménagé sur une surface interne de la partie proximale 40P de l'organe de préhension 40. Par ailleurs, pour chaque extracteur 46, le second organe de commande est formé par une saillie radiale 52 formant une rampe ménagée sur l'extracteur 46.

Du fait de cet agencement, pour chaque extracteur 46, les premier 50 et second 52 organes complémentaires de commande de cet extracteur 46 sont respectivement portés par l'organe de préhension 40 et l'extracteur 46. Plus particulièrement, ces premier 50 et second 52 organes de commande sont respectivement solidaires en translation de l'organe de préhension 40 et de l'extracteur 46.

En se référant plus particulièrement à la figure 7, on voit que l'organe de préhension 40 et le coulisseau 42 comprennent des moyens complémentaires 54 d'attelage entre eux autorisant le mouvement relatif d'accrochage entre cet organe de préhension 40 et le coulisseau 42.

Ces moyens complémentaires d'attelage 54 comprennent par exemple, comme cela est illustré sur la figure 7, une paire d'ergots d'attelage 56 sensiblement diamétralement opposés par rapport à l'axe X, emprisonnés axialement entre deux godrons d'attelage respectivement proximal 58 et distal 60 ménagés sur la surface interne de la partie proximale 40P en forme générale de manchon de l'organe de préhension 40. Les ergots d'attelage 56 sont déplaçables axialement entre les godrons proximal 58 et distal 60 de façon à autoriser le mouvement relatif d'accrochage des premier 50 et second 52 organes de commande de chaque extracteur 46.

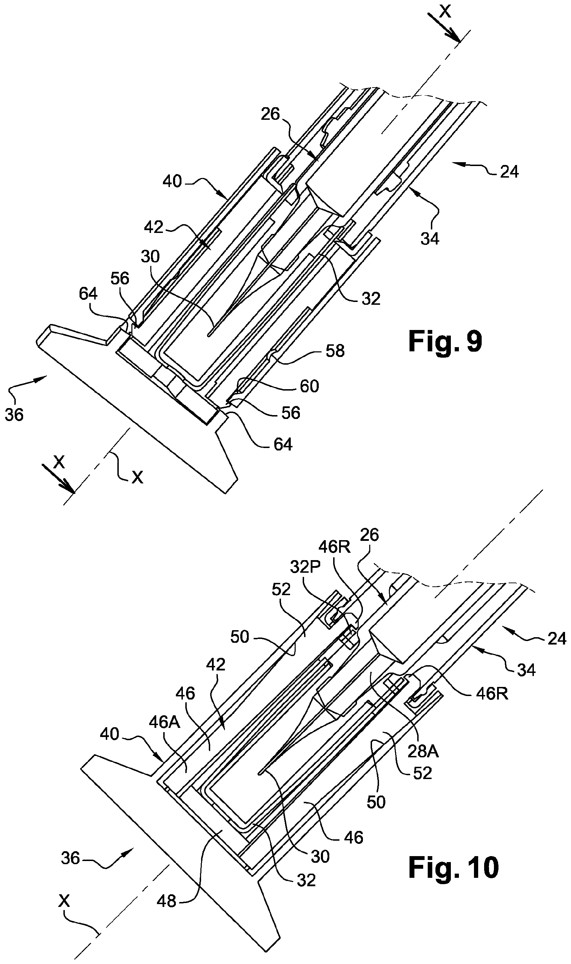

En se référant plus particulièrement aux figures 7 et 9, on voit que l'organe de préhension 40 et le coulisseau 42 comprennent des moyens complémentaires 62 de verrouillage entre eux activables à la fin du mouvement relatif d'accrochage.

Ces moyens complémentaires de verrouillage 62 comprennent par exemple, comme cela est illustré sur les figures 7 et 9, la paire d'ergots d'attelage 56 ainsi qu'une paire de fenêtres de verrouillage 64, diamétralement opposées par rapport à l'axe X, ménagées dans la partie proximale 40P de l'organe de préhension 40. Ces fenêtres de verrouillage 64 sont adjacentes au godron d'attelage distal 60. Ainsi, à la fin du mouvement relatif d'accrochage, les ergots d'attelage 56 du coulisseau 42 s'encliquettent dans les fenêtres de verrouillage 64 de l'organe de préhension 40 après franchissement du point dur matérialisé par le godron d'attelage distal 60.

Bien entendu, en variante, on pourrait ne prévoir qu'un seul ergot d'encliquetage 56 ou bien plus de deux ergots d'encliquetage 56 et une seule fenêtre de verrouillage 64 ou bien plus de deux fenêtres de verrouillage 64.

En se référant plus particulièrement aux figures 6 et 7, on voit que le support 34 et le coulisseau 42 comportent des butées complémentaires 66, 68 de positionnement de chaque extracteur 46 dans sa configuration de référence escamotée par rapport au capuchon de protection 32.

En se référant plus particulièrement aux figures 5 et 6, on voit que le coulisseau 42 comporte des saillies axiales internes 70 de centrage du capuchon de protection 32 dans le coulisseau 42.

On décrira ci-dessous les principales étapes de l'assemblage de l'ensemble d'injection 22 selon le premier mode de réalisation.

Initialement, on assemble séparément, d'une part, le support 34 de seringue d'injection 26 et, d'autre part, le dispositif de retrait 36.

Pour assembler le dispositif de retrait 36, on emboîte le coulisseau 42 dans la partie proximale 40P en forme générale de manchon de l'organe de préhension 40. Les ergots d'attelage 56 du coulisseau 42 franchissent le point dur formé par le godron d'attelage proximal 58 et s'intercalent axialement entre ce godron d'attelage proximal 58 et le godron d'attelage distal 60. L'organe de préhension 40 et le coulisseau 42 sont alors attelés entre eux de façon à autoriser un mouvement de translation relatif, correspondant au mouvement relatif d'accrochage, dont l'amplitude axiale est limitée par l'écartement axial entre les godrons d'attelage proximal 58 et distal 60.

Puis, selon un premier procédé d'assemblage de l'ensemble d'injection 22, on place la seringue d'injection 26 munie du capuchon de protection 32 recouvrant l'aiguille d'injection 30 de la seringue, dans le support 34 de seringue d'injection 26, comme cela est représenté sur la figure 2.

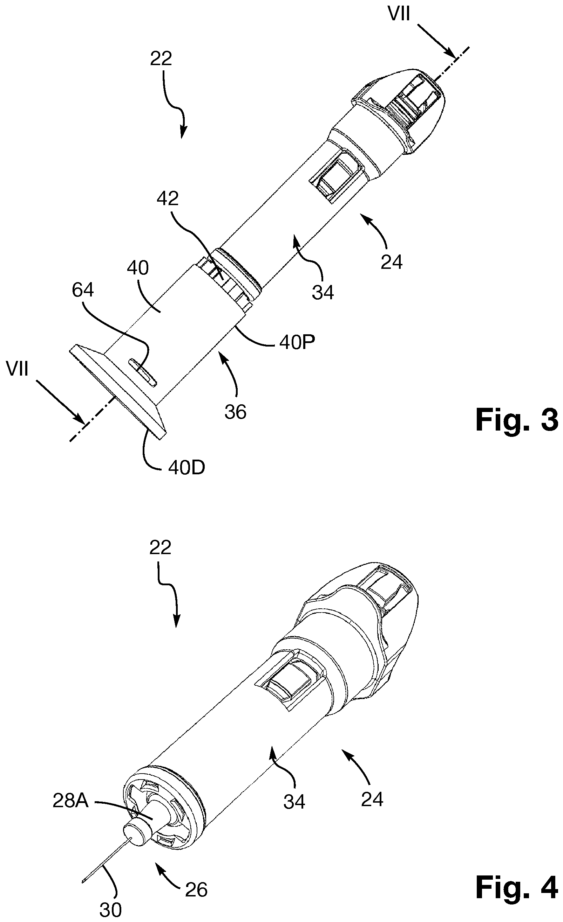

Ensuite, on place le dispositif de retrait 36 autour du capuchon de protection 32 en emboîtant le coulisseau 42 sur le capuchon de protection 32 par déplacement relatif axial parallèlement à l'axe X, jusqu'à ce que les butées complémentaires de positionnement 66, 68 coopèrent entre elles, comme cela est représenté sur les figures 3 et 7.

Chaque extracteur 46 est alors dans sa configuration de référence escamotée par rapport au capuchon de protection 32, comme cela est représenté sur la figure 8.

On notera que, selon un second procédé d'assemblage de l'ensemble d'injection 22, on peut placer la seringue d'injection 26 dans le support 34, non pas au moment décrit plus haut, mais lorsque le dispositif de retrait 36 se trouve dans la position illustrée sur les figures 3, 7 et 8. En effet, les extracteurs 46 sont alors chacun dans leur configuration de référence escamotée par rapport au capuchon de protection 32, permettant le passage de ce capuchon de protection 32 entre les extracteurs 46.

Le coulisseau 42 étant en butée contre le support 34 de seringue d'injection 26, on poursuit le déplacement axial parallèlement à l'axe X de l'organe de préhension 40 dans le sens de la flèche A illustrée sur la figure 8.

Comme les premier 50 et second 52 organes complémentaires de commande de chaque extracteur 46 sont respectivement portés par l'organe de préhension 40 et par cet extracteur 46, la poursuite du déplacement de l'organe de préhension 40 dans le sens de la flèche A provoque le mouvement relatif d'accrochage. Ainsi, les premier 50 et second 52 organes complémentaires de commande de chaque extracteur 46 coopérant entre eux par effet de rampe, chaque extracteur 46 évolue depuis sa configuration de référence escamotée, représentée sur la figure 8, vers sa configuration de coopération avec le capuchon de protection 32, représentée sur la figure 10.

On notera que la force, encore appelé puissance, exercée par effet de rampe sur chaque extracteur 46 s'applique, conformément à la répartition des forces dans un levier du troisième genre, entre l'extrémité de résistance 46R et l'extrémité d'appui 46A de l'extracteur 46.

Par ailleurs, à la fin du mouvement relatif d'accrochage, les ergots d'attelage 56 franchissent le point dur formé par le godron d'attelage distal 60 et s'emboîtent dans les fenêtres de verrouillage 64 de façon à verrouiller entre eux l'organe de préhension 40 et le coulisseau 42, comme cela est représenté sur les figures 1 et 9.

Le dispositif de retrait 36 est alors relié au support 34 de seringue d'injection 26. En effet, les crochets formant les extrémités de résistance 46R des extracteurs 46 sont en prise avec le bord d'extrémité proximal 32P du capuchon de protection 32, le dispositif de retrait 36 étant retenu sur le support 34 de seringue d'injection 26 par les forces de frottement maintenant le capuchon de protection 32 emboité sur la partie complémentaire 28A du corps 28 de seringue.

Lorsqu'un utilisateur souhaite utiliser l'ensemble d'injection 22, il s'empare de ce dernier dans la configuration représentée sur la figure 1, puis il tire l'organe de préhension 40 dans le sens de la flèche R illustrée sur cette figure 1, en appliquant sur cet organe de préhension 40 une force suffisante pour vaincre les forces de frottement maintenant le capuchon de protection 32 emboîté sur la partie complémentaire 28A du corps 28 de seringue.

Ce mouvement de manoeuvre appliqué par l'utilisateur à l'organe de préhension 40 entraîne le coulisseau 42, qui est verrouillé à cet organe de préhension 40, ainsi que le capuchon de protection 32 qui est entraîné par les extracteurs 46.

A la fin de ce mouvement de manoeuvre, le capuchon de protection 32 et complètement séparé de l'aiguille d'injection 30, l'ensemble d'injection 22 étant dans la configuration représentée sur la figure 4.

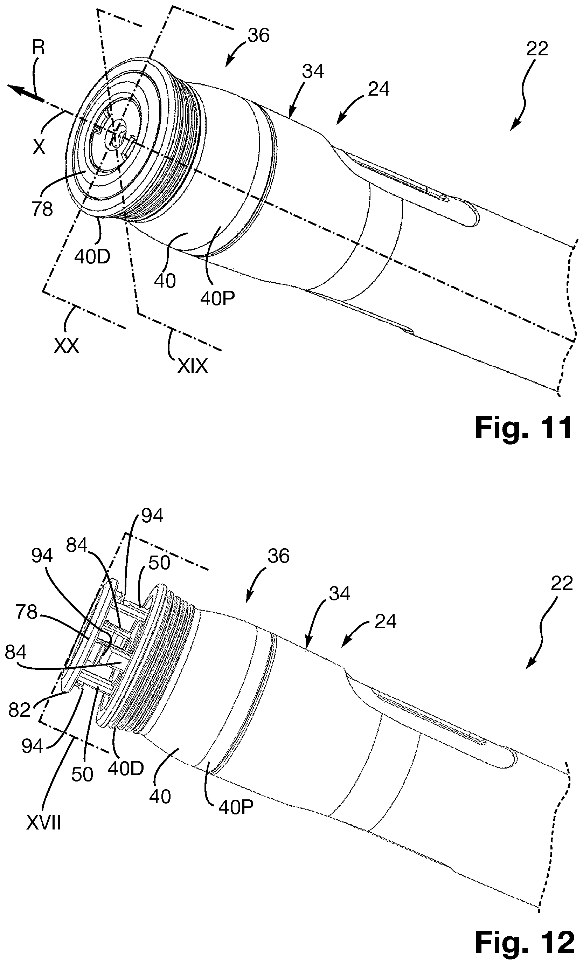

On décrira ci-dessous, en se référant aux figures 11 à 20, un ensemble d'injection 22 comprenant un dispositif 24 d'assistance au fonctionnement d'une seringue d'injection 26, selon un second mode de réalisation de l'invention. Comme dans les figures précédentes, l'ensemble d'injection 22 a une forme très générale de révolution autour de l'axe X.

Sur ces figures 11 à 20, les éléments analogues à ceux des figures précédentes sont désignés par des références identiques.

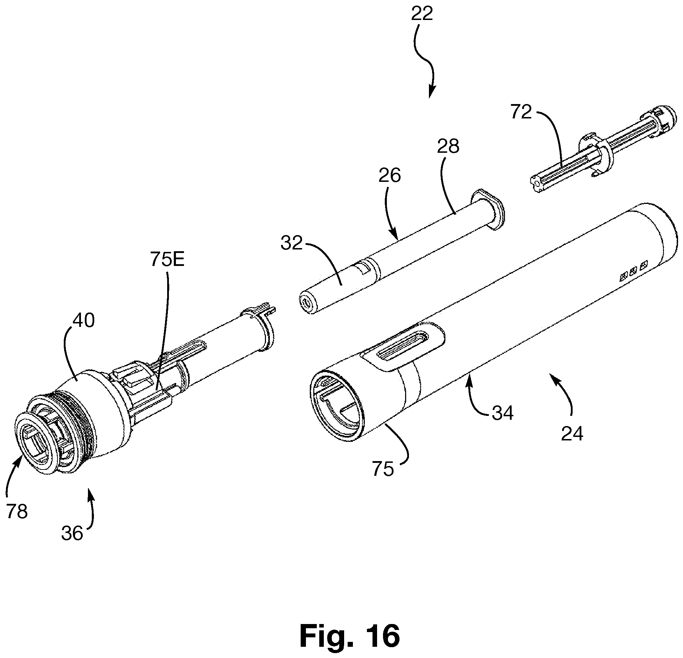

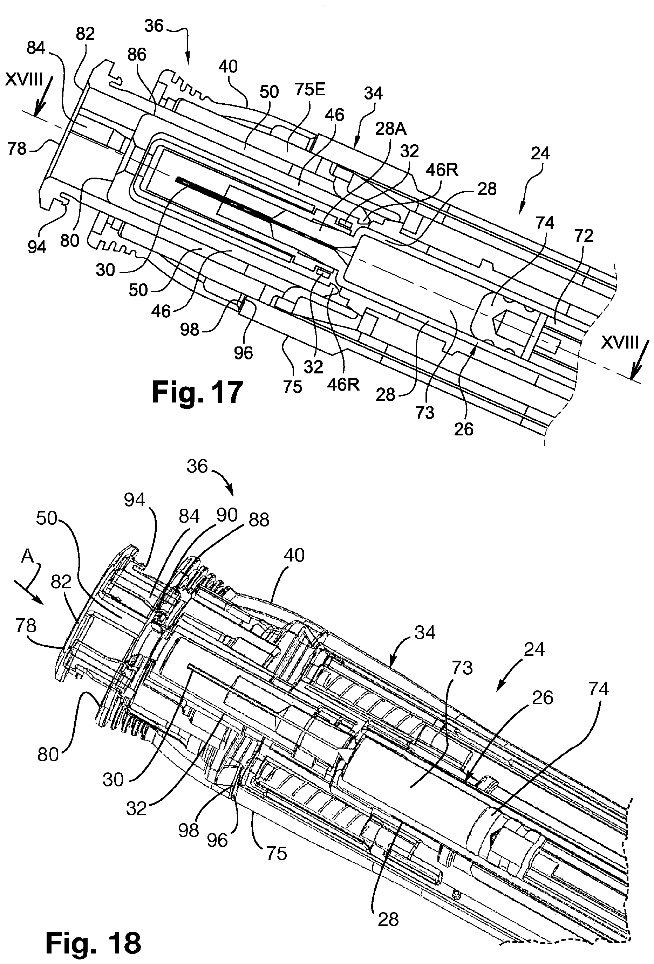

L'ensemble d'injection 22 représenté sur les figures 11 à 20 comprend une seringue d'injection 26, visible notamment sur les figures 16 à 20, analogue à celle décrite précédemment, logée dans le dispositif d'assistance 24 selon le second mode de réalisation de l'invention. On voit sur la figure 16 une tige 72 de manoeuvre d'un piston 74. Ce piston 74, visible sur les figures 17 à 20, est logé dans le corps 28 de seringue de façon à délimiter la chambre 73 contenant le médicament à injecter.

D'une part, le dispositif d'assistance 24 selon le second mode de réalisation permet d'empêcher ou de limiter les accidents de manipulation de la seringue d'injection 26, en masquant automatiquement, juste après l'injection, l'aiguille d'injection 30 de la seringue d'injection 26. D'autre part, ce dispositif d'assistance 24 selon le second mode de réalisation permet de rendre automatiques certaines étapes de fonctionnement de la seringue 26, notamment l'enfoncement de l'aiguille d'injection 30 de la seringue d'injection 26 dans le corps d'un patient et le déplacement distal du piston 74 de la seringue d'injection 26 dans le corps 28 de cette seringue d'injection 26.

Le support 34 de seringue du dispositif d'assistance 24 selon le second mode de réalisation comporte un corps externe 75 de forme générale tubulaire et un manchon d'extrémité 75E faisant saillie à une extrémité de ce corps externe 75.

Comme dans le premier mode de réalisation, le dispositif 36 de retrait du capuchon de protection 32 est relié de façon amovible au support 34 de seringue.

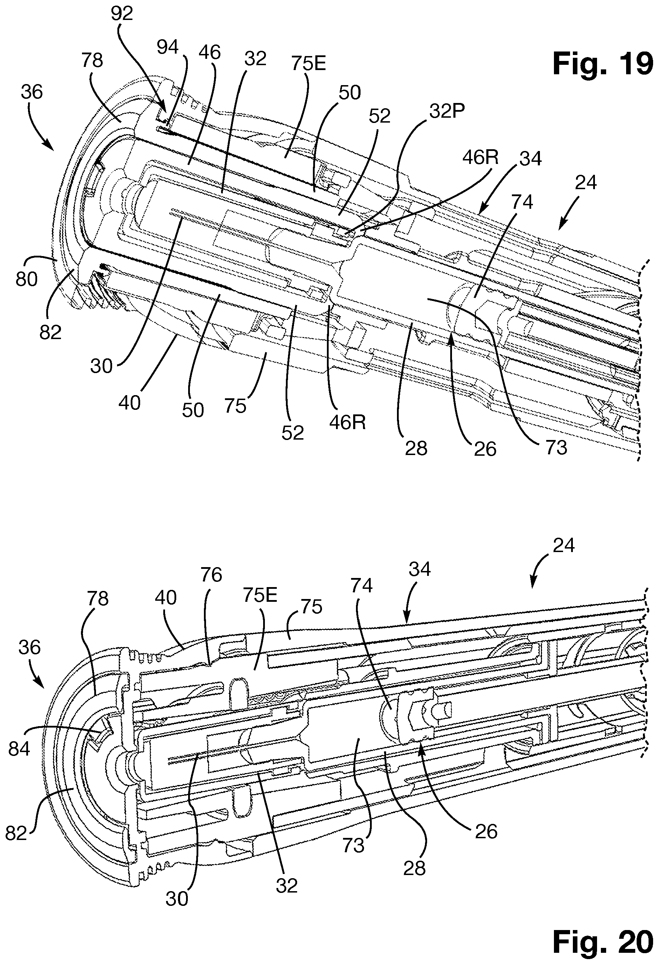

A cet effet, dans le second mode de réalisation, l'organe de préhension 40 et le support 34 de seringue comportent des moyens complémentaires 76 de verrouillage entre eux, visibles notamment sur la figure 20, libérables à l'initiation du mouvement de manoeuvre appliqué par l'utilisateur à l'organe de préhension 40 pour retirer le capuchon de protection 32 de l'aiguille d'injection 30.

Ces moyens complémentaires de verrouillage 76 sont formés, par exemple, pas des moyens complémentaires d'encliquetage ménagés sur l'organe de préhension 40 et le manchon d'extrémité 75E.

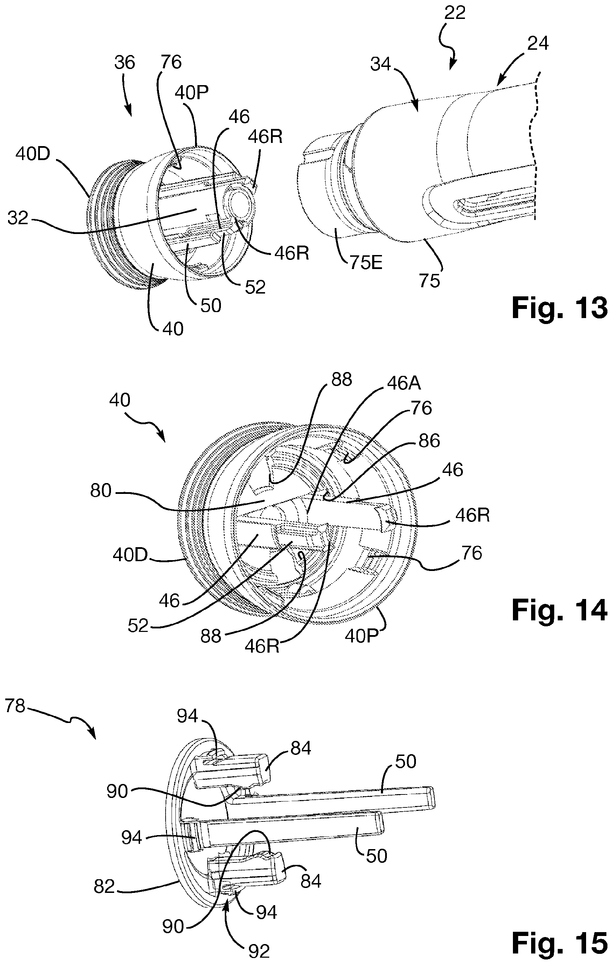

Comme dans le premier mode de réalisation, le dispositif de retrait 36 comprend l'organe de préhension 40. Toutefois, dans le second mode de réalisation, à la différence du premier mode de réalisation, chaque extracteur 46 est solidaire de l'organe de préhension 40, comme on peut le voir notamment sur la figure 14.

Plus particulièrement, l'organe de préhension 40 comprend deux extracteurs 46, sensiblement parallèles entre eux, destinés à coopérer avec le capuchon de protection 32 lors du retrait de ce capuchon de protection 32. En variante, l'organe de préhension 40 pourrait ne comporter qu'un seul extracteur 46 ou bien plus de deux extracteurs 46.

Par ailleurs, le dispositif de retrait 36 selon le second mode de réalisation comprend un organe de manoeuvre 78 (voir notamment figure 15) monté coulissant en translation, sensiblement parallèlement à l'axe X, dans l'organe de préhension 40.

Comme l'organe de préhension 40, l'organe de manoeuvre 78 est réalisé de préférence en plastique.

Le mouvement de manoeuvre appliqué par l'utilisateur à l'organe de préhension 40 pour retirer le capuchon de protection 32 de l'aiguille d'injection 30 est analogue au mouvement de manoeuvre décrit à propos du premier mode de réalisation de l'invention (voir flèche R sur la figure 11).

En se référant notamment aux figures 11 à 14, on voit que l'organe de préhension 40 comporte, comme dans le premier mode de réalisation, une partie proximale 40P, prolongée par une tête distale 40D dont la surface externe s'évase dans le sens proximal vers distal.

Comme dans le premier mode de réalisation de l'invention, chaque extracteur 46 est formé par un bras élastique s'étendant sensiblement longitudinalement dans la partie proximale 40P de l'organe de préhension 40.

L'organe de manœuvre 78 est monté coulissant en translation à travers une embase 80 formant une extrémité distale de la tête distale 40D de l'organe de préhension 40.

Comme dans le premier mode de réalisation, chaque extracteur 46 est formé par un bras élastique s'étendant sensiblement longitudinalement dans la partie proximale 40P de l'organe de préhension 40.

De préférence, comme dans le premier mode de réalisation, chaque extracteur 46 forme un levier du troisième genre déformable entre la configuration de référence escamotée et la configuration de coopération. Ainsi, d'une part, chaque extracteur 46 est muni d'une extrémité de résistance 46R, par exemple en forme de crochet, destinée à coopérer avec le bord d'extrémité proximal 32P du capuchon de protection 32. En variante, l'extrémité de résistance 46R de chaque extracteur 46 pourrait coopérer avec une autre surface du capuchon de protection 32. D'autre part, chaque extracteur 46 est muni d'une extrémité d'appui 46A, opposée à l'extrémité de résistance 46R, venue de matière avec l'embase 80 de l'organe de préhension 40 (voir figure 14). Ainsi, les extrémités d'appui 46A des extracteurs 46 sont reliées entre elles par l'embase 80 de l'organe de préhension 40.

Comme dans le premier mode de réalisation, les premier 50 et second 52 organes complémentaires de commande de chaque extracteur 46 coopèrent entre eux par effet de rampe en exécutant leur mouvement relatif d'accrochage. Toutefois, dans le second mode de réalisation, comme on peut le voir notamment sur les figures 13 à 15, pour chaque extracteur 46, le premier organe de commande 50 de cet extracteur 46 est porté par l'organe de manoeuvre 78 et le second organe de commande 52 de cet extracteur 46 est solidaire en translation de l'extracteur 46.

Ainsi, en se référant notamment aux figures 14 et 15, on voit que, pour chaque extracteur 46, le premier organe de commande est formé par un bras longitudinal 50 solidaire d'un anneau 82 formant une extrémité distale de l'organe de manoeuvre 78. L'organe de manoeuvre 78 comporte donc deux organes de commande 50, diamétralement opposés, sensiblement parallèles entre eux. Par ailleurs, pour chaque extracteur 46, le second organe de commande est formé par la saillie radiale 52 formant rampe ménagée sur l'extracteur 46.

En se référant notamment à la figure 15, on voit que l'organe de manoeuvre 78 comprend également deux doigts 84 sensiblement parallèles entre eux et aux premiers organes de commande 50, et solidaires de l'anneau 82. L'alignement des doigts 84 est perpendiculaire à l'alignement des premiers organes de commande 50.

Les premiers organes de commande 50 et les doigts 84 s'étendent à travers des orifices 86, 88 ménagés dans l'embase 80 de l'organe de préhension 40 (voir notamment figures 14, 17 et 18). En coopérant avec le contour de ces orifices 86, 88, les premiers organes de commande 50 et les doigts 84 sont guidés en translation dans l'organe de préhension 40.

En se référant notamment à la figure 18, on voit que l'organe de préhension 40 et l'organe de manoeuvre 78 comprennent des moyens complémentaires 90 de verrouillage entre eux libérables à l'initiation du mouvement relatif d'accrochage. Ces moyens complémentaires de verrouillage libérables 90 comprennent par exemple des moyens complémentaires à enfoncement et bossage complémentaires ménagés respectivement sur les doigts 84 et des bords des orifices 88 de passage et de guidage de ces doigts 84.

En se référant notamment aux figures 15 et 19, on voit que l'organe de préhension 40 et l'organe de manoeuvre 78 comprennent également des moyens complémentaires 92 de verrouillage entre eux activables à la fin du mouvement relatif d'accrochage. Ces moyens complémentaires de verrouillage 92 comprennent par exemple des moyens complémentaires d'encliquetage ménagés respectivement, d'une part, sur les extrémités de liaison avec l'anneau 82 des premiers organes de commande 50 et des doigts 84 et, d'autre part, sur les bords des orifices 86, 88 de passage et de guidage des premiers organes de commande 50 et des doigts 84. On a ainsi représenté, notamment sur les figures 15 et 19, des crochets d'encliquetage 94 solidaires des extrémités de liaison avec l'anneau 82 des premiers organes de commande 50 et des doigts 84.

En se référant plus particulièrement aux figures 17 à 20, on voit que l'organe de préhension 40 et le support 34 de seringue comportent des butées 96, 98 complémentaires assurant le positionnement de chaque extracteur 46 dans sa configuration de référence escamotée par rapport au capuchon 32.

Plus particulièrement, la butée 98 portée par le support de seringue 34 pour le positionnement d'un extracteur 46 dans sa configuration de référence escamotée est ménagée sur l'extrémité du corps externe 75 à travers laquelle fait saillie le manchon d'extrémité 75E.

On décrira ci-dessous des étapes possibles de l'assemblage de l'ensemble d'injection 22 illustré sur les figures 11 à 20.

Ainsi, on peut assembler le dispositif de retrait 36 de la façon suivante. Initialement, on place l'organe de manoeuvre 78 dans une position distale par rapport à l'organe de préhension 40, puis on emboîte les premiers organes de commande 50 et les doigts 84 de l'organe de manoeuvre 78 dans l'organe de préhension 40 en passant à travers les orifices 86, 88 ménagés dans cet organe de préhension 40, jusqu'à activer les moyens de verrouillage libérables 90. L'organe de manoeuvre 78 est alors positionné par rapport à l'organe de préhension 40 comme cela est illustré notamment sur les figures 12, 17 et 18.

On peut ensuite emboîter l'organe de préhension 40 sur le manchon d'extrémité 75E jusqu'à activer les moyens complémentaires de verrouillage 76 pour verrouiller entre eux l'organe de préhension 40 et le support 34 de seringue, comme cela est représenté notamment sur les figures 12, 17 et 18.

La seringue d'injection 26 peut être montée dans le support 34 avant ou après l'étape précédente, selon l'option d'assemblage choisie.

Par ailleurs, on peut également décider d'assembler l'organe de préhension 40 sur le manchon d'extrémité 75E avant d'avoir assemblé l'organe de manoeuvre 78 sur l'organe de préhension 40.

Une fois que l'on est dans la configuration illustrée sur les figures 17 et 18, la seringue d'injection 26 munie de son capuchon de protection 32 étant logée dans le support 34 de seringue, chaque extracteur 46 est dans sa configuration de référence escamotée par rapport au capuchon de protection 32. En effet, les butées complémentaires de positionnement 96, 98 ménagées sur l'organe de préhension 40 et l'extrémité du corps externe 75 coopèrent entre elles de façon à assurer que les extracteurs 46 soient dans leur configuration de référence escamotée par rapport au capuchon de protection 32.

Ensuite, on réalise le mouvement relatif d'accrochage en déplaçant l'organe de manoeuvre 78 dans le sens de la flèche A de la figure 18. A cet effet, on appuie sur l'anneau 82 de l'organe de manoeuvre 78 de façon à libérer les moyens de verrouillage libérables 90 et à enfoncer davantage l'organe de manoeuvre 78 dans le manchon de préhension 40.

Ainsi, les premier 50 et second 52 organes complémentaires de commande de chaque extracteur 46 coopérant entre eux par effet de rampe, chaque extracteur 46 évolue depuis sa configuration de référence escamotée, représentée sur la figure 17, vers sa configuration de coopération avec le capuchon de protection 32, représentée sur la figure 18.

A la fin du mouvement relatif d'accrochage, les moyens complémentaires de verrouillage 92 sont activés de façon à verrouiller entre eux l'organe de préhension 40 et l'organe de manoeuvre 78, comme cela est représenté sur la figure 19.

Lorsqu'un utilisateur souhaite utiliser l'ensemble d'injection 22 illustré sur les figures 11 à 20, il s'empare de ce dernier dans la configuration représentée sur la figure 11, puis il tire l'organe de préhension 40 dans le sens de la flèche R illustrée sur cette figure 11, en appliquant sur cet organe de préhension 40 une force suffisante pour, d'une part, libérer les moyens complémentaires de verrouillage 76 verrouillant entre eux l'organe de préhension 40 et le manchon d'extrémité 75E du support 34 de seringue et, d'autre part, vaincre les forces de frottement maintenant le capuchon de protection 32 emboîté sur la partie complémentaire 28A du corps 28 de seringue.

Ce mouvement de manoeuvre appliqué par l'utilisateur à l'organe de préhension 40 entraîne le capuchon de protection 32 qui est entraîné par les extracteurs 46.

A la fin de ce mouvement de manoeuvre, le capuchon de protection 32 est complètement séparé de l'aiguille d'injection 30, l'ensemble d'injection 22 étant dans la configuration représentée sur la figure 13.

On notera que, comme dans le premier mode de réalisation, dans ce second mode de réalisation, le mouvement relatif d'accrochage est indépendant du mouvement de manoeuvre appliqué par l'utilisateur à l'organe de préhension 40 lorsqu'il retire le capuchon de protection 32 de l'aiguille d'injection 30.

L'invention n'est pas limitée aux modes de réalisation présentés et d'autres modes de réalisation apparaîtront clairement à l'homme du métier. En particulier, la seringue d'injection peut être remplacée par une cartouche munie d'une aiguille.

## Revendications

1. Dispositif d'assistance (24) au fonctionnement d'une seringue d'injection (26) comprenant un corps (28) de seringue portant une aiguille d'injection (30) recouverte d'un capuchon de protection (32) amovible, le dispositif d'assistance (24) comprenant :
- un support (34) de seringue dans lequel le corps (28) de seringue est destiné à être logé, et
- un dispositif de retrait (36) du capuchon de protection (32), relié de façon amovible au support (34) de seringue, comprenant :
- un organe de préhension (40), destiné à être manoeuvré par un utilisateur pour retirer ce capuchon de protection (32) de l'aiguille d'injection (30), et
- au moins un extracteur (46) coopérant avec le capuchon de protection (32) lors du retrait du capuchon de protection (32),
**caractérisé en ce que** le dispositif de retrait (36) comprend des premier (50) et second (52) organes complémentaires de commande de l'extracteur (46) déplaçables l'un par rapport à l'autre selon un mouvement relatif de coopération entre eux, dit mouvement relatif d'accrochage, pour faire évoluer l'extracteur (46) entre une configuration de référence escamotée par rapport au capuchon de protection (32) et une configuration de coopération avec le capuchon de protection (32), le mouvement relatif d'accrochage étant indépendant du mouvement de manoeuvre appliqué par l'utilisateur à l'organe de préhension (40) pour retirer le capuchon de protection (32) de l'aiguille d'injection (30), et aussi **caractérisé en ce que** les premier (50) et second (52) organes complémentaires de commande de l'extracteur (46) coopèrent entre eux par effet de rampe sous l'effet du mouvement relatif d'accrochage.

2. Dispositif d'assistance (24) au fonctionnement d'une seringue d'injection (26) selon la revendication 1, dans lequel l'organe de préhension (40) comporte une partie proximale (40P) en forme générale de manchon, l'extracteur (46) étant formé par un bras élastique s'étendant sensiblement longitudinalement dans la partie proximale (40P) et formant un levier du troisième genre déformable entre les configurations de référence escamotée et de coopération, l'extracteur (46) étant muni d'une extrémité, dite de résistance (46R), destinée à coopérer avec le capuchon de protection (32).

3. Dispositif d'assistance (24) au fonctionnement d'une seringue d'injection (26) selon la revendication 1 ou 2, dans lequel le dispositif de retrait (36) comprend au moins deux extracteurs (46) formant respectivement deux leviers du troisième genre, sensiblement parallèles entre eux, ces extracteurs (46) étant munis chacun d'une extrémité, dite de résistance (46R), destinée à coopérer avec le capuchon de protection (32), et d'une extrémité opposée à l'extrémité de résistance, dite d'appui (46A), les extrémités d'appui (46A) des extracteurs (46) étant reliées entre-elles.

4. Dispositif d'assistance (24) au fonctionnement d'une seringue d'injection (26) selon l'une quelconque des revendications 1 à 3, dans lequel le mouvement de manoeuvre appliqué par l'utilisateur à l'organe de préhension (40) pour retirer le capuchon de protection (32) de l'aiguille d'injection (30) comprend une translation, le mouvement relatif d'accrochage étant parallèle à cette translation.

5. Dispositif d'assistance (24) au fonctionnement d'une seringue d'injection (26) selon l'une quelconque des revendications 1 à 4, dans lequel les premier (50) et second (52) organes complémentaires de commande de l'extracteur (46) sont respectivement portés par l'organe de préhension (40) et par l'extracteur (46).

6. Dispositif d'assistance (24) au fonctionnement d'une seringue d'injection (26) selon la revendication 5, dans lequel l'extracteur (46) est porté par un coulisseau (42) monté coulissant en translation dans l'organe de préhension (40), les premier (50) et second (52) organes complémentaires de commande de l'extracteur (46) étant respectivement solidaires en translation de l'organe de préhension (40) et de l'extracteur (46).

7. Dispositif d'assistance (24) au fonctionnement d'une seringue d'injection (26) selon les revendications 2 et 6 prises ensemble, dans lequel le premier organe (50) de commande de l'extracteur (46) est ménagé sur une surface interne de la partie proximale (40P) et le deuxième (52) organe de commande de l'extracteur (46) est ménagé sur l'extracteur (46).

8. Dispositif d'assistance (24) au fonctionnement d'une seringue d'injection (26) selon la revendication 7, dans lequel le support (34) de seringue et le coulisseau (42) comportent des butées complémentaires (66, 68) de positionnement de l'extracteur (46) dans sa configuration de référence escamotée par rapport au capuchon de protection (32).

9. Dispositif d'assistance (24) au fonctionnement d'une seringue d'injection (26) selon l'une quelconque des revendications 6 à 8, dans lequel l'organe de préhension (40) et le coulisseau (42) comprennent des moyens complémentaires (54) d'attelage entre eux autorisant entre eux le mouvement relatif d'accrochage.

10. Dispositif d'assistance (24) au fonctionnement d'une seringue d'injection (26) selon l'une quelconque des revendications 6 à 9, dans lequel l'organe de préhension (40) et le coulisseau (42) comprennent des moyens complémentaires (62) de verrouillage entre eux activables à la fin du mouvement relatif d'accrochage.

11. Dispositif d'assistance (24) au fonctionnement d'une seringue d'injection (26) selon la revendication 5, dans lequel l'extracteur (46) est solidaire de l'organe de préhension (40), le premier (50) organe de commande de l'extracteur (46) étant porté par un organe de manoeuvre (78) monté coulissant en translation dans l'organe de préhension (40) et le second (50) organe de commande de l'extracteur (46) étant solidaire en translation de l'extracteur (46).

12. Dispositif d'assistance (24) au fonctionnement d'une seringue d'injection (26) selon la revendication 11, dans lequel l'organe de préhension (40) et le support (34) de seringue comportent des butées complémentaires (96, 98) de positionnement de l'extracteur (46) dans sa configuration de référence escamotée par rapport au capuchon de protection (32).

13. Dispositif d'assistance (24) au fonctionnement d'une seringue d'injection (26) selon la revendication 11 ou 12, dans lequel l'organe de préhension (40) et le support (34) de seringue comportent des moyens complémentaires (76) de verrouillage entre eux libérables à l'initiation du mouvement de manoeuvre appliqué par l'utilisateur à l'organe de préhension (40) pour retirer le capuchon de protection (32) de l'aiguille d'injection (30).

14. Dispositif d'assistance (24) au fonctionnement d'une seringue d'injection (26) selon les revendications 12 et 13 prises ensemble, dans lequel le support (34) de seringue comporte un corps externe (75) de forme générale tubulaire et un manchon d'extrémité (75E) faisant saillie à une extrémité de ce corps externe (75),
la butée (98) portée par le support (34) de seringue pour le positionnement de l'extracteur (46) dans sa configuration de référence escamotée par rapport au capuchon de protection (32) étant ménagée sur l'extrémité du corps externe (75), et
les moyens complémentaires (76) de verrouillage de l'organe de préhension (40) et du support (34) de seringue étant ménagés sur l'organe de préhension (40) et le manchon d'extrémité (75E).

15. Dispositif d'assistance (24) au fonctionnement d'une seringue d'injection (26) selon l'une quelconque des revendications 11 à 14, dans lequel l'organe de préhension (40) et l'organe de manoeuvre (78) portant le premier organe (50) de commande de l'extracteur (46) comprennent des moyens complémentaires (90) de verrouillage entre eux libérables à l'initiation du mouvement relatif d'accrochage.

16. Dispositif d'assistance (24) au fonctionnement d'une seringue d'injection (26) selon l'une quelconque des revendications 11 à 15, dans lequel l'organe de préhension (40) et l'organe de manoeuvre (78) portant le premier organe de commande (50) de l'extracteur (46) comprennent des moyens complémentaires (92) de verrouillage entre eux activables à la fin du mouvement relatif d'accrochage.

17. Ensemble d'injection comprenant un dispositif (24) d'assistance au fonctionnement d'une seringue d'injection et une seringue d'injection (26) logée dans ce dispositif d'assistance (24), **caractérisé en ce que** le dispositif (24) d'assistance au fonctionnement de la seringue d'injection (26) est selon l'une quelconque des revendications précédentes.

## Patentansprüche

1. Vorrichtung zur Unterstützung (24) der Wirkweise einer Injektionsspritze (26), umfassend einen Spritzenkörper (28), der eine Injektionsnadel (30) trägt, die von einer abnehmbaren Schutzkappe (32) abgedeckt ist, wobei die Vorrichtung zur Unterstützung (24) umfasst:
- einen Spritzenträger (34), in dem der Spritzenkörper (28) zur Aufnahme bestimmt ist, und
- eine Vorrichtung zum Abziehen (36) der Schutzkappe (32), die mit dem Spritzenträger (34) lösbar verbunden ist, umfassend
- ein Greiforgan (40), das dazu bestimmt ist, von einem Benutzer gehandhabt zu werden, um diese Schutzkappe (32) von der Injektionsnadel (30) abzuziehen, und
- wenigstens einen Extraktor (46), der mit der Schutzkappe (32) beim Abziehen der Schutzkappe (32) zusammenwirkt,
**dadurch gekennzeichnet, dass** die Vorrichtung zum Abziehen (36) ein komplementäres erstes Organ (50) und zweites Organ (52) zum Betätigen des Extraktors (46) umfasst, die relativ zueinander gemäß einer relativen Bewegung zum Zusammenwirken miteinander, so genannte relative Einhängebewegung, bewegbar sind, um den Extraktor (46) zwischen einer gegenüber der Schutzkappe (32) zurückgezogenen Referenzkonfiguration und einer mit der Schutzkappe (32) zusammenwirkenden Konfiguration zu verändern, wobei die relative Einhängebewegung unabhängig von der Handhabungsbewegung ist, die von dem Benutzer auf das Greiforgan (40) aufgebracht wird, um die Schutzkappe (32) von der Injektionsnadel (30) abzuziehen, und ferner **dadurch gekennzeichnet, dass** das komplementäre erste (50) und zweite (52) Organ zum Betätigen des Extraktors (46) durch Rampenwirkung unter der Einwirkung der relativen Einhängebewegung miteinander zusammenwirken.

2. Vorrichtung zur Unterstützung (24) der Wirkweise einer Injektionsspritze (26) nach Anspruch 1, wobei das Greiforgan (40) einen proximalen Teil (40P) in allgemeiner Hülsenform aufweist, wobei der Extraktor (46) von einem Federarm gebildet ist, der sich im Wesentlichen in Längsrichtung in dem proximalen Teil (40P) erstreckt und einen Hebel der dritten Art bildet, der zwischen der zurückgezogenen Referenzkonfiguration und der zusammenwirkenden Konfiguration verformbar ist, wobei der Extraktor (46) mit einem Ende, so genanntes Widerstandsende (46R), versehen ist, das für das Zusammenwirken mit der Schutzkappe (32) bestimmt ist.

3. Vorrichtung zur Unterstützung (24) der Wirkweise einer Injektionsspritze (26) nach Anspruch 1 oder 2, wobei die Abziehvorrichtung (36) wenigstens zwei Extraktoren (46) umfasst, die jeweils zwei Hebel der dritten Art bilden und zueinander im Wesentlichen parallel sind, wobei diese Extraktoren (46) jeder versehen sind mit einem Ende, so genanntes Widerstandsende (46R), das für das Zusammenwirken mit der Schutzkappe (32) bestimmt ist, und mit einem Ende, das dem Widerstandsende gegenüberliegt, so genanntes Abstützende (46A), wobei die Abstützenden (46A) der Extraktoren (46) miteinander verbunden sind.

4. Vorrichtung zur Unterstützung (24) der Wirkweise einer Injektionsspritze (26) nach einem der Ansprüche 1 bis 3, wobei die Handhabungsbewegung, die von dem Benutzer auf das Greiforgan (40) aufgebracht wird, um die Schutzkappe (32) von der Injektionsnadel (30) abzuziehen, eine Translation umfasst, wobei die relative Einhängebewegung zu dieser Translation parallel ist.

5. Vorrichtung zur Unterstützung (24) der Wirkweise einer Injektionsspritze (26) nach einem der Ansprüche 1 bis 4, wobei das komplementäre erste (50) und zweite (52) Organ zum Betätigen des Extraktors (46) jeweils von dem Greiforgan (40) und von dem Extraktor (46) getragen werden.

6. Vorrichtung zur Unterstützung (24) der Wirkweise einer Injektionsspritze (26) nach Anspruch 5, wobei der Extraktor (46) von einem Schiebeelement (42) getragen wird, das in dem Greiforgan (40) in Translation verschiebbar gelagert ist, wobei das komplementäre erste (50) und zweite (52) Organ zum Betätigen des Extraktors (46) jeweils mit dem Greiforgan (40) und mit dem Extraktor (46) translationsfest sind.

7. Vorrichtung zur Unterstützung (24) der Wirkweise einer Injektionsspritze (26) nach Anspruch 2 und 6 zusammen, wobei das erste Organ (50) zum Betätigen des Extraktors (46) auf einer inneren Fläche des proximalen Teils (40P) vorgesehen ist und das zweite Organ (52) zum Betätigen des Extraktors (46) auf dem Extraktor (46) vorgesehen ist.

8. Vorrichtung zur Unterstützung (24) der Wirkweise einer Injektionsspritze (26) nach Anspruch 7, wobei der Spritzenträger (34) und das Schiebeelement (42) komplementäre Anschläge (66, 68) aufweisen zum Positionieren des Extraktors (46) in seiner gegenüber der Schutzkappe (32) zurückgezogenen Referenzkonfiguration.

9. Vorrichtung zur Unterstützung (24) der Wirkweise einer Injektionsspritze (26) nach einem der Ansprüche 6 bis 8, wobei das Greiforgan (40) und das Schiebeelement (42) komplementäre Mittel (54) zum Kuppeln miteinander umfassen, die zwischen einander die relative Einhängebewegung gestatten.

10. Vorrichtung zur Unterstützung (24) der Wirkweise einer Injektionsspritze (26) nach einem der Ansprüche 6 bis 9, wobei das Greiforgan (40) und das Schiebeelement (42) komplementäre Mittel (62) zum Verriegeln miteinander umfassen, die am Ende der relativen Einhängebewegung aktivierbar sind.

11. Vorrichtung zur Unterstützung (24) der Wirkweise einer Injektionsspritze (26) nach Anspruch 5, wobei der Extraktor (46) mit dem Greiforgan (40) fest verbunden ist, wobei das erste Organ (50) zum Betätigen des Extraktors (46) von einem Handhabungsorgan (78) getragen wird, das in dem Greiforgan (40) in Translation verschiebbar gelagert ist, und wobei das zweite Organ (52) zum Betätigen des Extraktors (46) mit dem Extraktor (46) translationsfest verbunden ist.

12. Vorrichtung zur Unterstützung (24) der Wirkweise einer Injektionsspritze (26) nach Anspruch 11, wobei das Greiforgan (40) und der Spritzenträger (34) komplementäre Anschläge (96, 98) aufweisen zum Positionieren des Extraktors (46) in seiner gegenüber der Schutzkappe (32) zurückgezogenen Referenzkonfiguration.

13. Vorrichtung zur Unterstützung (24) der Wirkweise einer Injektionsspritze (26) nach Anspruch 11 oder 12, wobei das Greiforgan (40) und der Spritzenträger (34) komplementäre Mittel (76) zum Verriegeln miteinander aufweisen, die zu Beginn der Handhabungsbewegung, die von dem Benutzer auf das Greiforgan (40) aufgebracht wird, um die Schutzkappe (32) von der Injektionsnadel (30) abzuziehen, lösbar sind.

14. Vorrichtung zur Unterstützung (24) der Wirkweise einer Injektionsspritze (26) nach Anspruch 12 und 13 zusammen, wobei der Spritzenträger (34) einen äußeren Körper (75) allgemeiner Röhrenform und eine Endhülse (75E), die an einem Ende dieses äußeren Körper (75) vorspringt, aufweist,
wobei der von dem Spritzenträger (34) getragene Anschlag (98) zum Positionieren des Extraktors (46) in seiner gegenüber der Schutzkappe (32) zurückgezogenen Referenzkonfiguration an dem Ende des äußeren Körper (75) vorgesehen ist, und
wobei die komplementären Mittel (76) zum Verriegeln des Greiforgans (40) und des Spritzenträger (34) auf dem Greiforgan (40) und der Endhülse (75E) vorgesehen sind.

15. Vorrichtung zur Unterstützung (24) der Wirkweise einer Injektionsspritze (26) nach einem der Ansprüche 11 bis 14, wobei das Greiforgan (40) und das Handhabungsorgan (78), die das erste Organ (50) zum Bestätigen des Extraktors (46) tragen, komplementäre Mittel (90) zum Verriegeln miteinander umfassen, die zu Beginn der relativen Einhängebewegung lösbar sind.

16. Vorrichtung zur Unterstützung (24) der Wirkweise einer Injektionsspritze (26) nach einem der Ansprüche 11 bis 15, wobei das Greiforgan (40) und das Handhabungsorgan (78), die das erste Organ (50) zum Bestätigen des Extraktor (46) tragen, komplementäre Mittel (92) zum Verriegeln miteinander umfassen, die am Ende der relativen Einhängebewegung aktivierbar sind.

17. Einspritzanordnung umfassend eine Vorrichtung zur Unterstützung (24) der Wirkweise einer Injektionsspritze und eine Injektionsspritze (26), die in dieser Vorrichtung zur Unterstützung (24) aufgenommen ist, **dadurch gekennzeichnet, dass** die Vorrichtung zur Unterstützung (24) der Wirkweise der Injektionsspritze (26) nach einem der vorhergehenden Ansprüche ausgeführt ist.

## Claims

1. Assistance device (24) for assisting in the operation of an injection syringe (26) comprising a syringe body (28) carrying an injection needle (30) covered with a removable protective cap (32), the assistance device (24) comprising:
- a syringe support (34) in which the syringe body (28) is intended to be housed, and
- a removal device (36) for removing the protective cap (32) removably connected to the syringe support (34), comprising:
- a gripping member (40), intended to be manoeuvred by a user in order to remove this protective cap (32) from the injection needle (30), and
- at least one extractor (46) cooperating with the protective cap (32) when removing the protective cap (32),
**characterised in that** the removal device (36) comprises first (50) and second (52) complementary control members for controlling the extractor (46) which can be moved relative to each other in a relative cooperation movement, known as a relative engagement movement, in order to move the extractor (46) between a reference configuration retracted relative to the protective cap (32) and a cooperation configuration cooperating with the protective cap (32), the relative engagement movement being independent of the manoeuvring movement applied by the user to the gripping member (40) in order to remove the protective cap (32) from the injection needle (30),
and characterised too
**in that** the first (50) and second (52) complementary control members for controlling the extractor (46) cooperate with each other by ramp effect under the effect of the relative engagement movement.

2. Assistance device (24) for assisting in the operation of an injection syringe (26) according to claim 1, wherein the gripping member (40) comprises a generally sleeve-shaped proximal part (40P), the extractor (46) consisting of an elastic arm extending substantially longitudinally in the proximal part (40P) and forming a class 3 lever deformable between the retracted reference and cooperation configurations, the extractor (46) being provided with a so-called resistance end (46R), intended to cooperate with the protective cap (32).

3. Assistance device (24) for assisting in the operation of an injection syringe (26) according to claim 1 or 2, wherein the removal device (36) comprises at least two extractors (46) forming respectively two class 3 levers, substantially parallel to each other, these extractors (46) each being provided with a so-called resistance end (46R), intended to cooperate with the protective cap (32), and a so-called support end (46A) opposite the resistance end, the support ends (46A) of the extractors (46) being connected together.

4. Assistance device (24) for assisting in the operation of an injection syringe (26) according to any one of claims 1 to 3, wherein the manoeuvring movement applied by the user to the gripping member (40) in order to remove the protective cap (32) from the injection needle (30) comprises a translation, the relative engagement movement being parallel to this translation.

5. Assistance device (24) for assisting in the operation of an injection syringe (26) according to any one of claims 1 to 4, wherein the first (50) and second (52) complementary control members for controlling the extractor (46) are carried respectively by the gripping member (40) and by the extractor (46).

6. Assistance device (24) for assisting in the operation of an injection syringe (26) according to claim 5, wherein the extractor (46) is carried by a slide (42) mounted slidably in translation in the gripping member (40), the first (50) and second (52) complementary control members for controlling the extractor (46) being attached respectively in translation to the gripping member (40) and to the extractor (46).

7. Assistance device (24) for assisting in the operation of an injection syringe (26) according to claims 2 and 6 taken together, wherein the first control member (50) for controlling the extractor (46) is formed on an inner surface of the proximal part (40P) and the second control member (52) for controlling the extractor (46) is formed on the extractor (46).

8. Assistance device (24) for assisting in the operation of an injection syringe (26) according to claim 7, wherein the syringe support (34) and the slide (42) comprise complementary stops (66, 68) for positioning the extractor (46) in its reference configuration retracted relative to the protective cap (32).

9. Assistance device (24) for assisting in the operation of an injection syringe (26) according to any one of claims 6 to 8, wherein the gripping member (40) and the slide (42) comprise complementary means (54) for coupling to each other allowing the relative engagement movement between them.

10. Assistance device (24) for assisting in the operation of an injection syringe (26) according to any one of claims 6 to 9, wherein the gripping member (40) and the slide (42) comprise complementary means (62) for locking to each other that can be activated at the end of the relative engagement movement.

11. Assistance device (24) for assisting in the operation of an injection syringe (26) according to claim 5, wherein the extractor (46) is attached to the gripping member (40), the first control member (50) for controlling the extractor (46) being carried by a manoeuvring member (78) mounted slidably in translation in the gripping member (40) and the second control member (52) for controlling the extractor (46) being attached in translation to the extractor (46).

12. Assistance device (24) for assisting in the operation of an injection syringe (26) according to claim 11, wherein the gripping member (40) and the syringe support (34) comprise complementary stops (96, 98) for positioning the extractor (46) in its reference configuration retracted relative to the protective cap (32).

13. Assistance device (24) for assisting in the operation of an injection syringe (26) according to claim 11 or 12, wherein the gripping member (40) and the syringe support (34) comprise complementary means (76) for locking to each other that can be released when starting the manoeuvring movement applied by the user to the gripping member (40) in order to remove the protective cap (32) from the injection needle (30).

14. Assistance device (24) for assisting in the operation of an injection syringe (26) according to claims 12 and 13 taken together, wherein the syringe support (34) comprises an outer body (75) of generally tubular shape and an end sleeve (75E) projecting from one end of this outer body (75),
the stop (98) carried by the syringe support (34) to position the extractor (46) in its reference configuration retracted relative to the protective cap (32) being formed on the end of the outer body (75), and
the complementary means (76) for locking the gripping member (40) and the syringe support (34) being formed on the gripping member (40) and the end sleeve (75E).

15. Assistance device (24) for assisting in the operation of an injection syringe (26) according to any one of claims 11 to 14, wherein the gripping member (40) and the manoeuvring member (78) carrying the first control member (50) for controlling the extractor (46) comprise complementary means (90) for locking to each other that can be released when starting the relative engagement movement.

16. Assistance device (24) for assisting in the operation of an injection syringe (26) according to any one of claims 11 to 15, wherein the gripping member (40) and the manoeuvring member (78) carrying the first control member (50) for controlling the extractor (46) comprise complementary means (92) for locking to each other that can be activated at the end of the relative engagement movement.

17. Injection assembly comprising an assistance device (24) for assisting in the operation of an injection syringe and an injection syringe (26) housed in this assistance device (24), **characterised in that** the assistance device (24) for assisting in the operation of the injection syringe (26) is according to any one of the preceding claims.
